(19) Europäisches Patentamt
European Patent Office
Office européen des brevets

(11) **EP 4 446 405 A1**

(12) **EUROPEAN PATENT APPLICATION**
published in accordance with Art. 153(4) EPC

(43) Date of publication:
**16.10.2024 Bulletin 2024/42**

(21) Application number: **22904138.9**

(22) Date of filing: **01.12.2022**

(51) International Patent Classification (IPC):
**C12N 1/20** (2006.01)    **C12M 1/26** (2006.01)
**C12N 7/02** (2006.01)    **B01J 20/24** (2006.01)
**B01J 20/28** (2006.01)

(52) Cooperative Patent Classification (CPC):
**C12M 1/26; C12N 1/20; C12N 7/02; B01J 20/24;
B01J 20/28**

(86) International application number:
**PCT/JP2022/044415**

(87) International publication number:
**WO 2023/106207 (15.06.2023 Gazette 2023/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **06.12.2021   JP 2021198032
03.03.2022   JP 2022032806**

(71) Applicant: **Asahi Kasei Kabushiki Kaisha
Tokyo 1000006 (JP)**

(72) Inventors:
• **FUKUSHIMA, Masakazu
Tokyo 100-0006 (JP)**

• **ODA, Kotaro
Tokyo 100-0006 (JP)**
• **ARAKAWA, Kazuhiko
Tokyo 100-0006 (JP)**
• **HARANO, Kodai
Tokyo 100-0006 (JP)**
• **SANO, Ayae
Tokyo 100-0006 (JP)**
• **HARA, Yuichi
Tokyo 100-0006 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **MICROORGANISM CONCENTRATION METHOD AND CONCENTRATION VESSEL**

(57)    Provided are a concentration method and vessel which make it possible to quickly obtain a highly concentrated product of bacteria or the like by using a simple means. The present invention pertains to a method for concentrating a microorganism in a sample liquid which contains a microorganism in a solvent by using porous cellulose fine particles which have a plurality of voids therein, said method comprising a step for adhering the microorganism to the surface of the porous cellulose fine particles by contacting the sample liquid to the porous cellulose fine particles, and a liquid removal step for concentrating the microorganism in the voids of the porous cellulose fine particles by removing the solvent of the sample liquid, wherein: the allowed water content C, which is the mass in tare weight of PBS(-) in swollen porous cellulose fine particles obtained by swelling 100 mg of dry particles in PBS(-) until the PBS(-) has stopped falling therefrom, is 1.9 g or higher; and the free water ratio, which is obtained by first obtaining the drying amount (C-E) obtained by subtracting the mass E of the PBS(-) after centrifuging the swollen porous cellulose fine particles for 1 minute at 1000G from said allowed water content C, and then dividing said drying amount by E, is 2.9 or higher. The present invention also pertains to a vessel.

**Description**

FIELD

The present invention relates to a concentration method for concentrating microorganisms in a solvent, such as microorganisms in a sample solution containing bacteria or viruses, for example, using porous cellulose microparticles having numerous interior pores, as well as a concentration vessel using the concentration method.

BACKGROUND

Diagnosis of infectious disease requires detection, identification and/or measurement of pathogens or quality-impairing harmful microorganisms in samples by rapid and convenient methods during environmental inspection or food microbial testing.

For example, pathogenic organisms and viruses can be identified by POCT (simple rapid testing), used to carry out early and proper clinical diagnosis by a physician or nurse by the patient's side for infectious disease diagnosis. For environmental inspection and food microbial testing as well, examination is carried out to detect harmful microorganisms including environmental indicator bacteria, among which are general bacterial and *E. coli* group pathogens, and food poisoning bacteria and viruses, for the purpose of employee or environmental hygiene management and quality control of raw materials, intermediate products and final products. When used by small businesses or for on-site inspection, all such testing requires specialized devices and convenient methods that can be performed without knowledge of microbiology.

Microbial examination methods can largely be divided into culturing methods in which a microorganism is identified based on biochemical properties using isolation culture, gene methods in which a gene specific for a microorganism is amplified by PCR or LAMP, and immunization methods which carry out detection by specific reaction between a microorganism-specific antigen marker and an antibody.

Because these examination methods all use small samples of about 0.01 to 1 mL, they utilize only a very small portion of the content of a detected substance (an actual bacteria or virus, or its surface or internal components) present in about 0.01 to 1 L, which is an amount that can be collected from a patient specimen (such as urine or body fluid), a food sample (such as a liquid sample or a bacterial or viral extract from a Stomacher[R]), or an environmental sample (for water quality, for example). This may restrict the detection accuracy or sensitivity in gene methods and immunization methods used for simple rapid testing.

In the case of trace microorganisms, long-term enrichment culture (24 hours, for example) is carried out to avoid false negatives in sampling, but this impacts the convenience and speed of the examination.

To solve these problems and to ensure adequate detection of substances without using large-scale equipment, adsorption carriers are used for concentration of microorganisms by the process of adsorption, isolation and collection.

In PTL 1, for example, inorganic microparticles (an amorphous metal silicate) and a sample are shaken for 1 to 20 minutes for adsorption of *E. coli*. However, the particles described in PTL 1 are not crosslinked cellulose particles and require shaking for 10 minutes or longer to obtain an adsorption rate of 50% or greater, using 10 mg of inorganic microparticles for 10 mL of sample containing *E. coli* at 1e3 cfu/mL (where "1eM" represents 10 raised to the Mth power, same hereunder). PTL 1 also contains no mention of the permissible water content per mass or the porosity of the dry particles.

PTL 2 describes a method in which aminosilica is contacted with a specimen containing a mixture of pathogenic bacteria and cells as an examination for bacterial mastitis, whereby the pathogenic bacteria alone are collected. However, the particles described in PTL 2 are not crosslinked cellulose particles, and an hour is required for stirring of the aminosilica and sample. Moreover, since the purpose is not concentration of the pathogenic bacteria but rather isolation of the cells, nothing is mentioned regarding the permissible water content per mass or the porosity of the dry particles.

PTL 3 describes an adsorption separation agent for viruses and cells using calcium phosphate-based porous granules having a two-type continuous pore structure, including continuous fine pores with a mean pore size of 20 to 500 nm and continuous small pores with a mean pore size of 1 to 50 $\mu$m. However, the particles described in PTL 3 are not crosslinked cellulose particles, and nothing is mentioned regarding the permissible water content per mass or the porosity of the dry particles.

PTL 4 describes a method for concentrating viruses, which includes a step of adding particles having cationic groups on the particle surfaces and having particle sizes of 0.5 to 300 $\mu$m, and an antibody for the surface antigen of the target virus, to a sample that potentially contains a virus, in order to adhere the virus onto the particles, and a step of separating and collecting the virus-adhered particles from the sample. It is stated that this method for concentrating viruses allows multiple specimens to be conveniently and simultaneously treated by simple means, while also being suitable for automation, and allowing concentration of a virus to a level that does not adversely affect nucleic acid amplification testing. However, the particles described in the publication are of a non-porous synthetic polymer obtained by polymerization of different monomers, while nothing is mentioned regarding applying the method for concentration of bacteria, or regarding the permissible water content per mass of the dry particles, or the porosity.

PTL 5 describes a method for removing viruses from a virus-containing sample, by contacting the virus-containing sample with an adsorbent having a structure comprising polyethyleneimine bonded to a porous particle-containing base carrier. Although crosslinked cellulose particles having a specific ligand are mentioned as the base carrier, there is no mention regarding application to concentration of bacteria, or regarding the permissible water content per mass of the dry particles, or the porosity.

PTL 6 describes industrial use of polycation-modified porous cellulose microparticles as a microcarrier for culturing of bacterial cells, yeast or animal and plant cells, but this assumes their adhesion and proliferation, while the publication does not mention a liquid removal step with removal of the solvent or concentration of the microorganisms in the pores, nor does it mention the permissible water content per mass of the dry particles or the porosity.

[CITATION LIST]

[PATENT LITERATURE]

[PTL 1] Japanese Patent Publication No. 5972174
[PTL 2] Japanese Patent Publication No. 4889263
[PTL 3] Japanese Patent Publication No. 2543766
[PTL 4] Japanese Patent Publication No. 4161167
[PTL 5] Japanese Unexamined Patent Publication No. 2019-194547
[PTL 6] Japanese Unexamined Patent Publication HEI No. 4-91142

SUMMARY

[TECHNICAL PROBLEM]

While PTLs 1, 2, 3 and 4 describe adsorbents for cells or viruses, their materials are non-porous synthetic polymers, porous amorphous metal silicates or porous calcium phosphate, rather than cellulose microparticles with high porosity and transmittance, which have the permissible water content per mass of the dry particles and the porosity as explained herein below, and therefore with such adsorbents it is difficult to obtain high-density masses of microorganisms.

Moreover, while PTL 5 describes polyethyleneimine-bonded crosslinked cellulose particles as the adsorbent, this type of adsorbent has a specific polyethyleneimine bonded as the ligand and is therefore specialized for adsorption removal of viruses.

In addition, even though PTLs 1, 2, 4 and 5 specify the conditions for contacting the adsorbent with a microorganic sample, they require at least 10 minutes of stirring and shaking to fully adsorb 10 mL of specimen sample onto the adsorbent, which reduces the simplicity of operation for such methods.

In light of the current situation of the prior art, the problem to be solved by the invention is to provide a microorganism concentration method that can rapidly produce a high concentrate of a microorganism by simple means, as well as a concentration vessel to be used in the concentration method.

[SOLUTION TO PROBLEM]

As a result of ardent research with the goal of solving the aforementioned problem, the present inventors have found, unexpectedly, that the problem can be solved by using porous cellulose microparticles as the adsorbent wherein, when 100 mg of dry particles is swelled with Phosphate-buffered saline(-) (hereunder also referred to as PBS(-)), the permissible water content C is 1.9 or greater, as the mass of PBS(-) in the porous cellulose microparticles in a swelled state such that PBS(-) does not fall by its own weight, and the free water ratio ($(C - E)/E$) is 2.9 or greater, as calculated by dividing the dehydration amount ($C - E$) (obtained by subtracting the mass E of the PBS(-) after centrifugation of the swelled porous cellulose microparticles for 1 minute at 1000 G from the permissible water content C) by E, and the invention has been completed on the basis of this finding.

Specifically, the present invention is as follows.

[1] A microorganism concentration method wherein a microorganism in a sample liquid comprising the microorganism in a solvent is concentrated using porous cellulose microparticles having numerous interior pores, the method including the following steps:

a step of contacting the sample liquid with the porous cellulose microparticles to adsorb the microorganism in the sample liquid onto the surfaces of the porous cellulose microparticles, and

a liquid removal step in which the solvent of the sample liquid is removed to concentrate the microorganism on the surface and/or in the pores of the porous cellulose microparticles; wherein when 100 mg of the dry porous cellulose microparticles is swelled with PBS(-), the permissible water content C is 1.9 g or greater, as the mass of PBS(-) in the porous cellulose microparticles in a swelled state such that PBS(-) does not fall by its own weight, and the free water ratio ((C - E)/E) is 2.9 or greater, as calculated by dividing the dehydration amount (C - E) (obtained by subtracting the mass E of the PBS(-) after centrifugation of the swelled porous cellulose microparticles for 1 minute at 1000 G from the permissible water content C) by E.

[2] The method according to [1] above, which further includes the following step:

a step in which, after the liquid removal step, the microorganism concentrated on the surface and/or in the pores of the porous cellulose microparticles using an eluent is recovered in the eluent.

[3] The method according to [1] or [2] above, which further includes a step between each of the steps, in which the porous cellulose microparticles are washed with a liquid or gas.

[4] The method according to any one of [1] to [3] above, wherein the mean particle size of the porous cellulose microparticles is 100 $\mu$m to 1000 $\mu$m.

[5] The method according to any one of [1] to [4] above, wherein the mean pore size of the pores in the porous cellulose microparticles is 1 $\mu$m to 100 $\mu$m.

[6] The method according to any one of [1] to [5] above, wherein the porous cellulose microparticles form a continuous pore structure with adjacent pores mutually communicating by openings in the membranes dividing them.

[7] The method according to any one of [1] to [6] above, wherein the surface area of the porous cellulose microparticles per 1 g of dry particles (the specific surface area) is 0.3 $m^2$/g to 4.4 $m^2$/g.

[8] The method according to any one of [1] to [7] above, wherein the cellulose composing the porous cellulose microparticles has a cationic functional group, and the charge capacity is 0.5 mmol/g to 5.0 mmol/g.

[9] The method according to [8] above, wherein the cationic functional group is at least one selected from the group consisting of primary amino, secondary amino and tertiary amino groups.

[10] The method according to [9] above, wherein the cationic functional group is N,N-diethylaminoethyl (DEAE).

[11] The method according to any one of [1] to [10] above, wherein the cellulose composing the porous cellulose microparticles is crosslinked between the molecules.

[12] The method according to any one of [1] to [11] above, wherein the cellulose composing the porous cellulose microparticles is made of copper ammonia regenerated cellulose.

[13] The method according to any one of [1] to [12] above, wherein the microorganism is a bacterium or virus.

[14] A concentration vessel to be used in a method in which a microorganism in a sample liquid comprising the microorganism in a solvent is concentrated using porous cellulose microparticles having numerous interior pores, wherein the concentration vessel comprises:

a cylindrical body having an inlet opening and an outlet opening;
a filter provided on the outlet opening side; and
porous cellulose microparticles filled into the spaces on the filter;

wherein

the sample liquid supplied through the inlet opening side is contacted with the porous cellulose microparticles, and the microorganism in the sample liquid is adsorbed onto the surfaces of the porous cellulose microparticles while the solvent of the sample liquid is removed from the outlet opening side, thereby concentrating the microorganism inside the pores of the porous cellulose microparticles, and

when 100 mg of the dry porous cellulose microparticles is swelled with PBS(-), the permissible water content C is 1.9 g or greater, as the mass of PBS(-) in the porous cellulose microparticles in a swelled state such that PBS(-) does not fall by its own weight, and the free water ratio ((C - E)/E) is 2.9 or greater, as calculated by dividing the dehydration amount (C - E) (obtained by subtracting the mass E of the PBS(-) after centrifugation of the swelled porous cellulose microparticles for 1 minute at 1000 G from the permissible water content C) by E.

[15] The concentration vessel according to [14] above, wherein the mean particle size of the porous cellulose microparticles is 100 $\mu$m to 1000 $\mu$m.

[16] The concentration vessel according to [14] or [15] above, wherein the mean pore size of the pores in the porous cellulose microparticles is 1 $\mu$m to 100 $\mu$m.

[17] The concentration vessel according to any one of [14] to [16] above, wherein the porous cellulose microparticles form a continuous pore structure with adjacent pores mutually communicating by openings in the membranes dividing them.

[18] The concentration vessel according to any one of [14] to [17] above, wherein the surface area of the porous cellulose microparticles per 1 g of dry particles (the specific surface area) is 0.3 m$^2$/g to 4.4 m$^2$/g.

[19] The concentration vessel according to any one of [14] to [18] above, wherein the cellulose composing the porous cellulose microparticles has a cationic functional group, and the charge capacity is 0.5 mmol/g to 5.0 mmol/g.

[20] The concentration vessel according to [19] above, wherein the cationic functional group is at least one selected from the group consisting of primary amino, secondary amino and tertiary amino groups.

[21] The method according to [20] above, wherein the cationic functional group is N,N-diethylaminoethyl (DEAE).

[22] The concentration vessel according to any one of [14] to [21] above, wherein the cellulose composing the porous cellulose microparticles is crosslinked between the molecules.

[23] The concentration vessel according to any one of [14] to [22] above, wherein the cellulose composing the porous cellulose microparticles is made of copper ammonia regenerated cellulose.

[24] The concentration vessel according to any one of [14] to [23] above, wherein the microorganism is a bacterium or virus.

[25] A microorganism concentration method wherein a microorganism in a sample liquid comprising the microorganism in a solvent is concentrated using porous cellulose microparticles having numerous interior pores, the method including the following steps:

a step of contacting the sample liquid with the porous cellulose microparticles in a concentration vessel comprising a cylindrical body having an inlet opening and an outlet opening; a filter provided on the outlet opening side; and porous cellulose microparticles filled into the spaces on the filter, to adsorb the microorganism in the sample liquid onto the surfaces of the porous cellulose microparticles, and

a liquid removal step in which aeration is carried out and the solvent of the sample liquid is removed to concentrate the microorganism on the surface and/or in the pores of the porous cellulose microparticles;

wherein when 100 mg of the dry porous cellulose microparticles is swelled with PBS(-), the permissible water content C is 1.9 g or greater, as the mass of PBS(-) in the porous cellulose microparticles in a swelled state such that PBS(-) does not fall by its own weight, and the free water ratio ((C - E)/E) is 2.9 or greater, as calculated by dividing the dehydration amount (C - E) (obtained by subtracting the mass E of the PBS(-) after centrifugation of the swelled porous cellulose microparticles for 1 minute at 1000 G from the permissible water content C) by E.

[26] The microorganism concentration method according to [25] above, wherein the aeration is aeration with air using a syringe from the inlet opening part of the concentration vessel.

[27] The microorganism concentration method according to [26] above, wherein the volume of the air is 0.5 mL to 1 mL per 1 mg of the porous cellulose microparticles.

[ADVANTAGEOUS EFFECTS OF INVENTION]

With the concentration method and concentration vessel of the invention it is possible to concentrate a microorganism using specific porous cellulose microparticles having a high porosity and dehydration rate as indicated by the permissible water content and free water ratio, to allow high adsorption of the microorganism per dry weight of the particles and obtain a high-density mass of the microorganism, and also to collect the microorganism at high-concentration from the high-density mass of the microorganism. The concentration method and concentration vessel of the invention can therefore be suitably used for microbial examination.

BRIEF DESCRIPTION OF DRAWINGS

Fig. 1 is a set of photographs showing porous cellulose microparticles (crosslinked cellulose) and other concentrating carriers.

Fig. 2 is a conceptual drawing that illustrates the concept of "permissible water content" and "free water ratio", and how they are determined.

Fig. 3 is a photograph showing porous cellulose microparticles (particles of the invention) and particle suspensions for Comparative Examples (1) to (4).

Fig. 4 is an electron microscope photograph of a porous cellulose microparticle after adsorption of *E. coli*.

Fig. 5 is a schematic diagram of a concentration vessel for holding porous cellulose microparticles.

DESCRIPTION OF EMBODIMENTS

The invention will now be explained in detail by embodiments.

The first embodiment of the invention is a microorganism concentration method wherein a microorganism in a sample liquid comprising the microorganism in a solvent is concentrated using porous cellulose microparticles having numerous interior pores, the method including the following steps:

a step of contacting the sample liquid with the porous cellulose microparticles to adsorb the microorganism in the sample liquid onto the surfaces of the porous cellulose microparticles, and
a liquid removal step in which the solvent of the sample liquid is removed to concentrate the microorganism on the surface and/or in the pores of the porous cellulose microparticles;

wherein when 100 mg of the dry porous cellulose microparticles is swelled with PBS(-), the permissible water content C is 1.9 g or greater, as the mass of PBS(-) in the porous cellulose microparticles in a swelled state such that PBS(-) does not fall by its own weight, and the free water ratio ((C - E)/E) is 2.9 or greater, as calculated by dividing the dehydration amount (C - E) (obtained by subtracting the mass E of the PBS(-) after centrifugation of the swelled porous cellulose microparticles for 1 minute at 1000 G from the permissible water content C) by E.

[Permissible water content (g) and free water ratio]

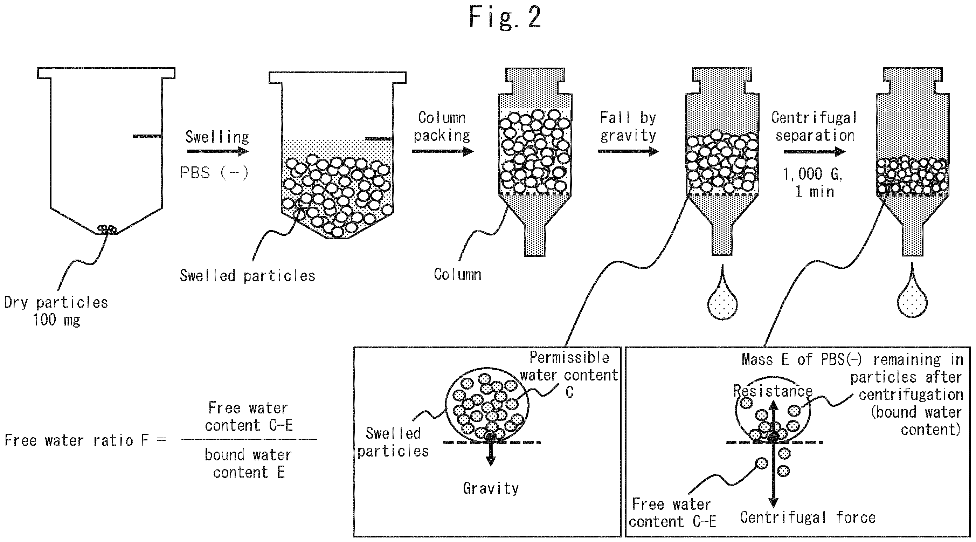

Fig. 2 shows a conceptual drawing illustrating the concept of "permissible water content" and "free water ratio", and how they are determined.

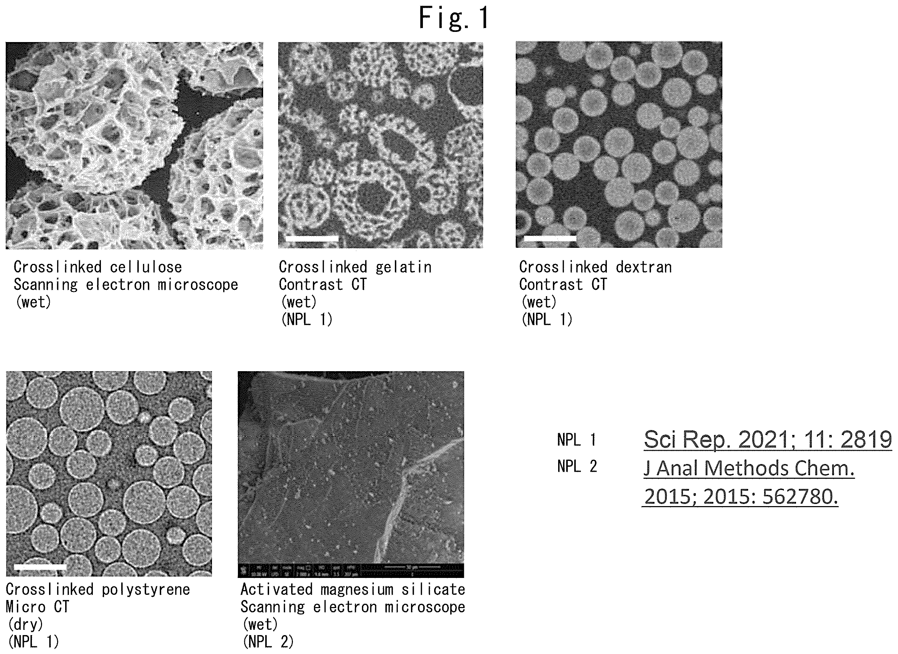

The permissible water content is the mass of PBS(-) in the porous cellulose microparticles that are in a swelled state such that PBS(-) does not fall by its own weight when 100 mg of the dry particles is swelled with PBS(-), and it is an absolute indicator of how much liquid will be absorbed by the particles. Since a larger value results in more formation of voids inside the particles by swelling, a high permissible water content means that the sizes of the pores during swelling are large and the porosity is high. It is generally known that higher porosity facilitates permeation of water in a porous substance, and a higher permissible water content means higher permeation of water. Large pore sizes allow microorganisms to be adsorbed on the interior surfaces of the pores, and high water permeability allows microorganisms to penetrate into the pores. This also aids elution (desorption) of microorganisms during their collection. It is preferred for the permissible water content to be high, being preferably 1.9 g or higher, more preferably 2.1 g or higher, even more preferably 2.4 g or higher and most preferably 2.6 g or higher. The porous cellulose microparticles ("crosslinked cellulose") used for the embodiment shown in Fig. 1 have more numerous pores inside the microparticles during swelling compared to the other concentrating carriers, showing their high porosity.

The free water ratio is a relative indicator of how much liquid is removed by the swelled particles, being represented as the ratio ((C - E)/E) between the mass of PBS(-) removed from the particles (the permissible water content C in the swelled state minus the mass of PBS(-) remaining in the particles after centrifugal force of 1000 G has been applied for 1 minute (E: bound water content)) (C - E: free water content), and E. A larger value means a greater free water content with respect to the bound water content in the particles, or in other words, a greater relative dehydration amount. A large dehydration amount corresponds to high elimination of excess water, allowing a high-density mass of microorganisms (number of adsorbed microorganisms per weight of the particles (number/mg)) to be obtained. It is preferred for the free water ratio to be high, being preferably 2.9 or higher, more preferably 5.7 or higher, even more preferably 6.4 or higher and most preferably 7.1 or higher.

Removal of the water during the liquid removal step may be accomplished by a publicly known method. Examples of such methods include, but are not limited to, reduced pressure drying, centrifugal separation, substitution by aeration with air, for example, and removal using a water absorbent, which may be selected as appropriate for the purpose. For example, centrifugal separation is appropriate when the force of water removal is to be controlled, and substitution by aeration with air using a syringe or the like is appropriate when the water is to be removed in a simple manner without using a specialized device. According to one embodiment, centrifugal separation for 1 minute at 1000 G corresponds to aeration with 1 to 2 mL of air.

The type of cellulose composing the porous cellulose microparticles for this embodiment is not particularly restricted and may be any publicly known cellulose such as copper ammonia regenerated cellulose, viscose regenerated cellulose, cotton, pulp or polynosic, among which copper ammonia regenerated cellulose and viscose regenerated cellulose are preferred, and copper ammonia regenerated cellulose is more preferred. Copper ammonia regenerated cellulose has a low degree of crystallinity and is therefore highly reactive during functional group modification, allowing homogeneous modification even throughout the interiors of porous particles, and resulting in excellent shape stability of the particles.

[Mean particle size (μm)]

The mean particle size of the porous cellulose microparticles of this embodiment is preferably 100 μm to 1000 μm, more preferably 100 μm to 500 μm, even more preferably 200 μm to 400 μm and yet more preferably 200 μm to 280 μm. If the mean particle size is smaller than 100 μm the aperture of the filters on the inlet opening side and outlet opening side of the concentration vessel will need to be smaller to hold the porous cellulose microparticles, and this will increase the pressure loss when supplying the sample liquid containing the microorganism or when removing the solvent of the sample liquid, thus reducing usability and restricting the amount of processing liquid. If the mean particle size is greater than 1000 μm, on the other hand, resistance on the particles will increase, producing greater flow in the gaps between the particles than inside the particles, often resulting in less uniform (or lower) water permeability. For the purpose of the present specification, the mean particle size is the average particle diameter measured for at least 20 particles, after removing the excess water once the porous particles have been swelled with water or physiological saline, and setting an appropriate magnification with an optical microscope.

[Mean pore size of pores (μm)]

The pore size of the porous cellulose microparticles of this embodiment, as the mean pore size, is preferably 1 μm to 100 μm, more preferably 10 μm to 80 μm and even more preferably 10 μm to 50 μm. A mean pore size smaller than 1 μm may impede free movement of the sample liquid comprising the microorganism in the porous cellulose microparticles. Since viruses generally have a particle size of approximately 0.02 μm while bacteria have a particle size of approximately 1 μm to 5 μm, the very small particle sizes of viruses means that Brownian motion is their dominant movement (displacement) in pores, whereas bacteria have more dominant particle sizes, and consequently if the mean pore size of the porous cellulose microparticles is smaller than 1 μm this may significantly limit adsorption of all types of microorganism inside the particles. If the mean pore size is larger than 100 μm, on the other hand, it will not be possible to ensure adequate surface area for adsorption of microorganisms, potentially preventing efficient concentration of the microorganisms. For the purpose of the present specification, the mean pore size is the average pore size as the distance between membranes measured for at least 20 particles, after removing the excess water once the porous particles have been swelled with water or physiological saline, and setting an appropriate magnification with an electron microscope. The pore size for a pore that was not spherical or a true circle was defined as its shortest diameter.

The porous cellulose microparticles of the embodiment preferably form a continuous pore structure with the pores mutually communicating by openings in the membranes dividing them. Such a mutually communicating structure allows microorganisms to be efficiently adsorbed through to the interiors of the porous cellulose microparticles, thereby further increasing the surface area for adsorption of microorganisms.

[Specific surface area (m²/g)]

The surface area (specific surface area) of the dry porous cellulose microparticles of this embodiment is preferably 0.3 m²/g to 4.4 m²/g, more preferably 0.6 m²/g to 3.3 m²/g and even more preferably 0.8 m²/g to 1.7 m²/g. A surface area of smaller than 0.3 m²/g will have less of the surface required for adsorption of microorganisms, potentially preventing efficient concentration of the microorganisms. A dry surface area of 4.4 m²/g or greater, on the other hand, will lower the charge per unit area and may lower the adhesion rate of microorganisms. The surface area, for the purpose of the present specification, is the surface area measured by the BET method.

[Charge capacity (mmol/g)]

For the charge capacity of the porous cellulose microparticles of the embodiment it is necessary to take into account the effect of the hydroxyl groups in the cellulose base, unlike particles of polystyrene or gelatin. It is also necessary to consider intramolecular hydrogen bonding and crystallinity, both of which differ considerably from polysaccharide particles such as dextran particles. The charge capacity of the porous particles of the embodiment is preferably 0.5 mmol/g to 5.0 mmol/g, more preferably 0.5 mmol/g to 3.0 mmol/g, even more preferably 0.6 mmol/g to 3.0 mmol/g, yet more preferably 0.6 mmol/g to 2.5 mmol/g, even yet more preferably 0.7 mmol/g to 2.5 mmol/g, and most preferably 0.9 mmol/g to 2.0 mmol/g. If the charge capacity is within this range, the charge per unit surface area will be larger, commensurate with the specified range for the surface area of the pores, thereby improving the adhesion rate for microorganisms. This will also allow the microorganisms to be collected without interference due to charge when they are collected using an eluent. The charge capacity, for the present purpose, is the average charge capacity measured for at least 3 samples, by adsorbing chlorine onto the amine groups of the porous particles using hydrochloric acid, and then using a sodium sulfate solution to wash off the adsorbed chlorine and titrating it with silver nitrate using potassium chromate as the indicator.

The cationic substituent in the porous cellulose microparticles of the embodiment is not particularly restricted, but it is preferably at least one type selected from the group consisting of primary amino, secondary amino and tertiary amino groups, more preferably tertiary amino groups, and even more preferably diethylaminoethyl groups.

The porous cellulose microparticles of the embodiment can be produced by a method of molding a solution of cellulose into a desired shape while cooling it to below the solidification temperature for freezing, and then either removing the solvent by extraction or inactivating its dissolution power. The freezing temperature is generally preferred to be set no lower than 40°C below the freezing temperature of the solvent, and will usually be selected in a range of 0 to 20°C lower than the freezing temperature.

The frozen cellulose solution or cellulose derivative solution then has the cellulose- or cellulose derivative-dissolving solvent removed by extraction, or else its solubility is lowered (both of these treatments may also be collectively referred to herein as "solvent removal"), to obtain a solidified cellulose porous body. The conditions for solvent removal are not particularly restricted. It is usually sufficient to rapidly introduce the frozen body into an arbitrary coagulating bath or regenerating bath, with the coagulating bath or regenerating bath preferably being at no higher than the freezing temperature of the solution. A cellulose derivative, however, requires a cellulose regeneration step, where the regeneration is carried out simultaneously with or successively with (i.e. after) the solvent removal. The regeneration itself may be carried out by a common method. Using the production method described above does not require introduction of extraneous materials such as porous materials into the polymer solution during production, and it therefore allows droplets with fine uniform diameters to be easily created, for desired control of the particle sizes. The diameters and shapes of the pores are primarily determined by the sizes and shapes of the solvent crystals formed during freezing solidification of the solvent in the solution. The freezing solidification conditions including the type and temperature of polymer solution may therefore be altered to adjust the shapes and sizes of the pores.

Using this method, the resulting porous cellulose microparticles will form a continuous pore structure with the pores mutually communicating by openings in the membranes dividing them.

The porous cellulose microparticles used in the concentration method of the embodiment may be used directly as granulated cellulose particles, but they are preferably used after crosslinking. The crosslinking method is not restricted, and crosslinking between the cellulose porous body molecules may be accomplished using a crosslinking agent having two or more functional groups that can react and bond with the hydroxyl groups of cellulose. One example is a bifunctional organic substance. An example of such a substance is one of the form X-R-Z [where R represents an aliphatic residue containing a carbon atom, and X and Z are halogens or epoxy groups which are bonded to the carbon atoms of the aliphatic residues], which reacts relatively easily in the presence of an alkaline reactive substance. Examples of bifunctional compounds suitable for reaction include, but are not particularly limited to, epichlorhydrin, dichlorohydrin, 1,2- or 3,4-diepoxybutane, bisepoxypropyl ether, ethylene glycol-bis-epoxypropyl ether, 1,4-butanediol-bis-epoxypropyl ether, and their closely related compounds. Crosslinking allows the crystal structure of the cellulose to be maintained even with abundant modification with cationic substituents, so that the particle structure can be kept stable.

There are no particular restrictions on the method of modifying the cationic substituents on the cellulose composing the porous cellulose microparticles to be used in the concentration method of the embodiment, and introduction of groups may be by multiple methods including methods of direct modification of hydroxyl groups and methods of modification via functional groups with epoxy groups. A preferred method is direct modification of the hydroxyl groups of cellulose using 2-(diethylamino)ethyl chloride hydrochloride. Because of the high abundance of hydroxyl groups on cellulose, it is possible to achieve uniform modification of numerous cationic substituents throughout the interiors of the particles by direct modification of hydroxyl groups. With introduction via third substituents such as epoxy groups, on the other hand, the amount and variation of cationic substituents introduced depends on the amount of third substituents introduced, and it is difficult to control the amount of introduction throughout the particle interiors. There are no particular restrictions on the reaction conditions for introducing cationic substituents using 2-(diethylamino)ethyl chloride hydrochloride, and the charge density inside the pores can be controlled by setting the optimal pH in consideration of pH changes of the solution due to the 2-(diethylamino)ethyl chloride hydrochloride itself, or by including an additive such as sodium sulfate to minimize charge effects of the cellulose hydroxyl groups.

A preferred cationic functional group for this embodiment is N,N-diethylaminoethyl (DEAE).

[Dry particle weight (mg) per swelled volume (mL)]

The porous cellulose microparticles of the embodiment have a dry particle weight (mg) of preferably 70 mg or less, more preferably 55 mg or less and even more preferably 40 mg or less, per 1 mL of particle volume when swelled with PBS(-) (swelled volume). A low dry particle weight per swelled volume means that the porous particles have large sizes of pores in the liquid and high porosity. If the dry particle weight per swelled volume is within this range, the water permeability will be high and the microorganisms will be able to freely move within the pores, thereby allowing them to be efficiently adsorbed onto the inner surfaces of the pores. During collection of the microorganisms as well, mobility of microorganisms out of the pores after elution with an eluent is also increased, thus facilitating the elution of the microorganisms.

[Adsorption rate (%)]

In order to use as many detectable substances as possible in a specimen (bacteria or viruses themselves, or their surface or interior components), the porous cellulose microparticles to be used for the adsorption method of the embodiment preferably have a high adsorption rate of microorganisms on the surfaces of the porous cellulose microparticles, being preferably 50% or greater, more preferably 60% or greater, even more preferably 70% or greater and most preferably 80% or greater. Incidentally, the surfaces of the porous cellulose microparticles include both the outermost surfaces and pore surfaces of the microparticles.

[Amount of microorganism-containing sample (mL)]

The amount of sample containing microorganisms to be contacted in the adsorption method of the embodiment may be increased or decreased depending on the amount of porous cellulose microparticles used, but for the vortex method (the method of contacting the particles and microorganisms by uniform dispersion as described in Example 3) it is preferably 10 mL or less, more preferably 5 mL or less, even more preferably 1 mL or less and most preferably 0.1 mL or less, with respect to 2 mg of dry weight, since a greater liquid volume reduces the probability of contact between the particles and microorganisms while stirring. For the column method (the method of contacting the particles and microorganisms by solid phase separation as described in Example 4), on the other hand, the flow volume is not restricted but is preferably 0.1 mL to 10,000 mL, more preferably 1 mL to 10,000 mL and even more preferably 1 mL to 1000 mL, since a large amount of sample requires more time for liquid passage and complicates the operation.

A second embodiment of the invention is a concentration vessel to be used in a method in which a microorganism in a sample liquid comprising the microorganism in a solvent is concentrated using porous cellulose microparticles having numerous interior pores, wherein the concentration vessel comprises:

a cylindrical body having an inlet opening and an outlet opening;
two filters respectively provided on the inlet opening side and the outlet opening side across a predetermined gap; and
porous cellulose microparticles filled into the space defined by the two filters;

wherein

the sample liquid supplied through the inlet opening side is contacted with the porous cellulose microparticles, and the microorganism in the sample liquid is adsorbed onto the surfaces of the porous cellulose microparticles while the solvent of the sample liquid is removed from the outlet opening side, thereby concentrating the microorganism on the surface and/or in the pores of the porous cellulose microparticles, and
when 100 mg of the dry porous cellulose microparticles is swelled with PBS(-), the permissible water content C is 1.9 g or greater, as the mass of PBS(-) in the porous cellulose microparticles in a swelled state such that PBS(-) does not fall by its own weight, and the free water ratio ((C - E)/E) is 2.9 or greater, as calculated by dividing the dehydration amount (C - E) (obtained by subtracting the mass E of the PBS(-) after centrifugation of the swelled porous cellulose microparticles for 1 minute at 1000 G from the permissible water content C) by E.

The porous cellulose microparticles to be used for the second embodiment may be the same as those used for the first embodiment described above.

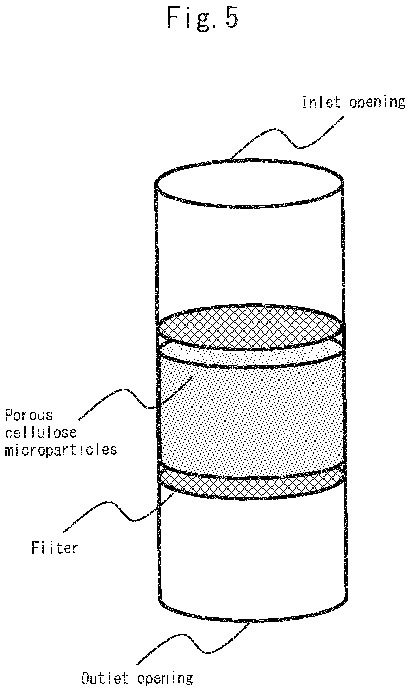

Fig. 5 shows a schematic diagram of the concentration vessel.

The term "microorganism" as used herein is not particularly restrictive and broadly encompasses various bacteria including *Escherichia coli, Staphylococcus aureus, Pseudomonas aeruginosa* and *Salmonella,* as well as viruses, fungi, protozoans and rickettsia. These microorganisms are the target of concentration for this embodiment. The phrase "sample liquid containing a bacteria or virus" is also not restrictive, and broadly encompasses patient specimens (such as urine and body fluids), food samples (such as liquid samples or bacterial or viral extracts obtained using a Stomacher[R]), and environmental samples (such as water quality samples).

The term "concentrating carrier" as used herein refers to a carrier to be used for concentration by adhering to a target to be concentrated from within a solution containing the target, and reducing the supernatant. The target of removal may be bacteria, viruses or proteins other than the target of concentration, and is not particularly restricted.

[Adsorption per particle weight (number/mg)]

As used herein, the phrase "adsorption per particle weight" refers to the number of microorganisms adsorbed per dry weight of the carrier (number/mg) after the microorganisms have been adsorbed, with a larger value indicating that a higher density mass of microorganisms has been obtained. The high-density mass of microorganisms may be used either directly on the carrier (complex) after adhesion of the microorganisms, or after separating the adsorption carrier and the microorganisms. The water content may also be removed from the high-density mass or left to remain.

[Concentration of microorganisms]

To obtain a concentrated bacterial solution as one example of the embodiment, the microorganisms alone may be collected by passing an eluent through the high-density mass. A smaller water content brought in from the high-density mass allows a higher-concentration concentrate to be obtained. Removal of the water can be accomplished by a publicly known method. Examples of such methods include, but are not limited to, reduced pressure drying, centrifugal separation, substitution by aeration with air, for example, and removal using a water absorbent, which may be selected as appropriate for the purpose. For example, centrifugal separation is appropriate when the force of water removal is to be controlled, and substitution by aeration with air using a syringe or the like is appropriate when the water is to be removed in a simple manner without using a specialized device. For this embodiment, centrifugal separation for 1 minute at 1000 G corresponds to aeration with 1 to 2 mL of air. An appropriate eluent may be selected for purposes requiring living bacterial solutions.
The eluent may be selected based on salt concentration, firstly, being preferably 0.1 M or greater and more preferably 0.3 M or greater, but since an excessively high salt concentration may adversely affect bacterial collection, it is also preferably 1 M or lower.
Secondly, the ion species of the eluent is selected to be a cation or anion, with higher valency generally corresponding to higher elution power, and selection of the ion species being important, since elution power is lost in the case of sodium borate, for example. In environments in which the present invention is to be carried out (such as 0°C to 40°C), on the other hand, it is necessary to consider whether the solubility allows an ion concentration in this range to be obtained. One preferred embodiment where this is taken into account is a salt including magnesium ion, and more preferably magnesium chloride.
Thirdly, the pH of the eluent is important for maintaining survival of microorganisms and for inducing electrostatic interaction by surface charge between the microorganisms and adsorption carrier, in a direction favorable for dissociation. A buffering solution may also be added to the eluent to maintain a consistent pH. According to one embodiment, a pH of 2 to 10 is preferred and a pH of 4 to 8 is more preferred for *Escherichia coli* and *Staphylococcus aureus.* However, the pH is not limited to this range and may be selected as appropriate for the type of microorganism. The eluent may also contain surfactants, enzymes, chaotropic salts, sugars, amino acids or polymers, depending on the form in which the collected bacteria are to be used.

[Concentration of microorganism components]

When components on the surface of or inside a concentrated bacteria or virus are to be obtained, as an example of another embodiment, a bacteriolytic component may be contacted with the high-density mass to collect the desired eluted components. A lower content of water brought in from the high-density mass can yield a higher concentration of desired components, and the method described above may be used for removal of the water. The bacteriolytic component is not restricted so long as it is appropriately selected for the type of bacterium or virus, but the excellent chemical strength (such as hydrolysis resistance and chemical resistance) of the porous cellulose microparticles may be utilized to include alkali solution, organic solvent, surfactant, enzyme, chaotropic salt, sugar, amino acid, or polymer components, for example. The excellent physical strength (such as heat resistance and abrasion resistance) of the porous cellulose microparticles may also be utilized to allow elution of desired components by ultrasonic or heat treatment of the high-density mass.

[Separation of contaminating components by washing]

The high-density mass of microorganisms may be washed with an appropriate solution in an optional step. One example of washing is for removal of unwanted contaminants in a patient specimen or food component. By selection of an appropriate eluent it is also possible to selectively remove specific types of microorganisms or cells. When the high-density mass is to be provided directly for examination (such as for detection of bacteria by an immunological method), washing may be carried out for B/F separation of the detection reagent to be used. The washing solution is not restricted and may be appropriately selected for the type of bacterium or virus, but a composition that does not elute or lyse the microorganism being examined is preferred in consideration of the range of selection of the eluent and/or bacteriolytic component.

EXAMPLES

The present invention will now be explained in more specific detail through the following Examples and Comparative Examples, with the understanding that the invention is in no way limited to the Examples.

First, the methods for measuring the physical properties of the structures and for preparing the bacterial solutions used in the Examples and Comparative Examples will be described.

(1) Permissible water content (g) and free water ratio

The tare weight A (g) of an empty column (Poly-Prep[R], Bio-Rad Laboratories, Inc.) is measured. A heat drying moisture meter (MX-50, A&D) is used to measure the water content of a sample of porous particles, and the porous particle sample is filled into a column to a dry weight of 100 mg. After adding 20 mL of PBS(-) (166-23555, FujiFilm-Wako) and allowing the PBS(-) to drop by gravity to the bottom of the column until it no longer falls, the column weight B (g) is measured. The permissible water content C (g) per 100 mg of particles is calculated by the following formula (1).

$$\text{Permissible water content C (g) per 100 mg of particles} = B - (0.1 + A) \quad \text{Formula (1)}$$

The column is then processed by centrifugal separation for 1 minute at 1000 G, and the column weight D (g) after dehydration by centrifugal force is measured. The mass of PBS(-) after centrifugation (E: bound water content) (g) per 100 mg of particles is calculated by the following formula (2).

$$\text{Bound water content E (g)} = D - (0.1 + A) \quad \text{Formula (2)}$$

The free water ratio F is calculated by the following formula (3).

$$F = (C - E)/E \quad \text{Formula (3)}$$

Fig. 2 shows a method of measuring the permissible water content (g) and free water ratio in overview.

(3) Mean particle size ($\mu$m)

The mean particle size is the average particle diameter measured for at least 20 particles, after removing the excess water once the porous particles have been swelled with water or physiological saline, and setting an appropriate magnification with a microscope.

(4) Mean pore size of pores ($\mu$m)

The mean pore size of the pores is the average pore size as the distance between membranes measured for at least 20 particles, after removing the excess water once the porous particles have been swelled with water or physiological saline, and setting an appropriate magnification with a microscope. The pore size for a pore that was not spherical or a true circle was defined as its shortest diameter.

(5) Specific surface area ($m^2$/g)

The surface area is that measured by the BET method, and the specific surface area is the surface area per 1 g of dry particles.

(6) Charge capacity

The sample is measured out to 5.0 g and a funnel and suction filter are used for washing twice with 30 mL of distilled water for exchange with water. The sample is then washed twice with 30 mL of a 0.3 N aqueous hydrochloric acid solution and 4 times with 50 mL of a $10^{-4}$ N aqueous hydrochloric acid solution, in that order. A funnel and suction filter are then used for washing 4 times with 50 mL of a 10% aqueous sodium sulfate solution, and the filtrate is collected. A 2 mL portion of a 0.1 N aqueous potassium chromate solution is added to the collected filtrate and the mixture is stirred. Titration is performed with a 1 N aqueous silver nitrate solution to determine the titer A (mL).

The sample that has completed washing with the 10% aqueous sodium sulfate solution in the previous step is separately washed 4 times with 50 mL of purified water, and then dried at 105°C for 15 hours or longer, after which the weight is measured with a balance to determine the dry weight B (g).

For measurement of the charge capacity, the measured titer G (mL) and the dry weight H (g) of the sample are used for calculation according to the following formula (4).

$$\text{Charge capacity (mmol/g)} = 1(\text{N})\,(\text{mol/L}) \times \text{G/H} \qquad \text{Formula (4)}$$

(7) Dry particle weight (mg) per swelled volume (mL)

A heat drying moisture meter (MX-50, A&D) is used to measure the water content of the porous particle sample, and the porous particle sample is filled into an empty column (Poly-Prep$^R$, Bio-Rad Laboratories, Inc.) to a dry weight of 100 mg. After adding 20 mL of PBS(-) and allowing the PBS(-) to drop by gravity to the bottom of the column, at the point where it no longer falls, the filled volume I (mL) of the particles is measured using the column scale. The dry particle weight per swelled volume J (mg/mL) is calculated by the following formula (5).

$$\text{Dry particle weight per swelled volume J (mg/mL)} = 100/\text{I} \qquad \text{Formula (5)}$$

(8) Method for preparing different bacterial solutions

(Preparation of *E. coli* solution)

A 15 $\mu$L portion of an *E. coli* strain (ATCC No.25922) is inoculated into 1 mL of autoclave-sterilized 3% Tripticase Soy Broth liquid medium (211768, BD Co.), and shake culturing is carried out for 16 hours at 37°C under aerobic conditions. Centrifugal separation is carried out at 2000 $\times$ g for 5 minutes, the supernatant liquid is removed, and 1 mL of distilled water is added, to obtain a bacterial solution with an OD (600 nm) of 1 (approximately 1e9 cfu/mL). The obtained *E. coli* solution is used after appropriate dilution with physiological saline.

(Preparation of *Staphylococcus aureus* solution)

A 15 $\mu$L portion of a *Staphylococcus aureus* strain (ATCC No.25923) is inoculated into 1 mL of autoclave-sterilized 3% Tripticase Soy Broth liquid medium (211768, BD Co.), and shake culturing is carried out for 16 hours at 37°C under aerobic conditions. Centrifugal separation is carried out at 2000 $\times$ g for 5 minutes, the supernatant liquid is removed, and 1 mL of distilled water is added, to obtain a bacterial solution with an OD (600 nm) of 1 (approximately 1e9 cfu/mL). The obtained *Staphylococcus aureus* solution is used after appropriate dilution with physiological saline.

(Preparation of *E. coli* T1 phage)

A 15 $\mu$L portion of an *E. coli* strain (JM109 No.25922) is inoculated into 10 mL of autoclave-sterilized 3% Tripticase Soy Broth liquid medium (211768, BD Co.), and shake culturing is carried out for 16 hours at 37°C under aerobic conditions, after which 100 $\mu$L of E. *coli* T1 phage (T1, NBRC20001) diluted with a 1% aqueous Proteose Peptone solution (Sigma, P0431-250G) is inoculated in and culturing is continued for 7 hours. After removing the bacterial cells from the culture solution using a 0.22 $\mu$m filter, the molecular weight components of ≤100 kDa were eliminated by ultrafiltration and the number of phage plaques was measured by the double agar plate method to be 3.7e9 pfu/mL. The obtained *E. coli* T1 phage solution is used after appropriate dilution with physiological saline.

(9) Preparation of porous cellulose microparticles and comparison particles

The names, suppliers and physical properties of the comparison particles (1) to (4) prepared for the Comparative Examples are shown in Table 1.

[Table 1]

| | Invention particles (a)-(d) | Comparison particles (1) | Comparison particles (2) | Comparison particles (3) | Comparison particles (4) |
|---|---|---|---|---|---|
| Name | Porous cellulose microparticles | Cultispher[R]-S | Cytodex[R]-1 | Fluorisil | DOWEX™ 1X8 |
| Manufacturer | - | M9043-10G/Sigma-Aldrich | 17044802/Cytiva | 065-05252/FujiFilm Wako | 327-97491/FujiFilm Wako |
| Material | Crosslinked cellulose | Crosslinked gelatin | Crosslinked dextran | Activated magnesium silicate | Crosslinked polystyrene |
| Modifying group | Diethylaminoethyl | Without | Diethylaminoethyl | Without | Trimethylbenzylammonium |
| Area-to-weight ratio (when dry) ($m^2$/g) | 1.1 | 0.4 | 0.44 | 289 | No data |
| Pore size (when wet) ($\mu$m) | 30 | 20 | Non-porous | 0.006 | Non-porous |
| Charge capacity (mmol/g) | 0.55~2.6 | 0.52 | 1.53 | Below detection limit | 3.2 |

[Preparation of porous cellulose microparticles (also referred to herein as "invention particles") (a)]

A cellulose-copper ammonium solution with a viscosity of 2 poise at 5°C, a cellulose concentration of 2%, a copper concentration of 1.2% and an ammonia concentration of 4%, prepared using refined linter as the starting material, was sprayed in an amount of 500 mL together with nitrogen gas using a two-fluid nozzle, and was used while being stirred in 10 L of silicon oil (SH200,1.5cs, Toray Co., Ltd.) that had been cooled to -40°C. The silicon oil was heated to -20°C while continuing stirring for 20 minutes, and then 10 L of 40% sulfuric acid that had been cooled to -35°C was loaded in. After loading the sulfuric acid, stirring was continued for 8 hours, the temperature was raised to 20°C, and the silicon oil was removed, extracting and washing off the remainder, to obtain porous cellulose microparticles. A sorting sieve was used to obtain a particle sample with a mean particle size of 200 $\mu$m to 280 $\mu$m.

The particle sample was then measured out to 6.66 g and loaded into the reaction mixture (purified water: 267.77 g, sodium dodecylsulfate: 0.016 g, 48% aqueous sodium hydroxide solution: 13.05 g, epichlorhydrin: 3.845 g), which was then stirred for 1 hour in a thermostatic bath at 60°C. The sample was washed 3 times with 8000 mL of purified water using suction filtration to obtain crosslinked cellulose microparticles.

The total amount of the obtained crosslinked cellulose microparticles was loaded into reaction mixture (purified water: 217.30 g, anhydrous sodium sulfate: 88.83 g, sodium dodecylsulfate: 0.016 g, 2-(diethylamino)ethylchloride HCl aqueous solution (50%): 11.08 g), which was then stirred. A reaction starting solution (48% aqueous sodium hydroxide solution: 5.33 g, purified water: 6.83 g) was then added and the mixture was gently stirred, after which reaction was conducted while stirring for 1 hour in a thermostatic bath at 70°C. After 1 hour, a neutralizing solution (36% hydrochloric acid: 3.14 g, purified water: 3.14 g) was loaded in, the mixture was stirred, and the sample was washed 3 times with 8000 mL of purified water using suction filtration to obtain the invention particles. The obtained particles had a mean particle size of 240 $\mu$m, a mean pore size of 30 $\mu$m, through-holes with a mean pore size of 30 $\mu$m or smaller, a surface area per 1 g of dry particles (specific surface area) of 1.1 $m^2/g$ and a charge capacity of 1.80 mmol/g. The porous cellulose microparticles obtained in this manner were labeled as (a).

[Preparation of porous cellulose microparticles (b) to (d) with different charge capacities]

After obtaining crosslinked cellulose microparticles by the same procedure as described above, 6.66 g of the particles was loaded into the reaction mixture (purified water: 217.30 g, anhydrous sodium sulfate: 88.83 g, sodium dodecylsulfate: 0.016 g, 2-(diethylamino)ethyl chloride hydrochloride solution (50%): 2.325 g), which was then stirred. A reaction starting solution (48% aqueous sodium hydroxide solution: 1.930 g, purified water: 6.83 g) was then added and the mixture was gently stirred, after which reaction was conducted while stirring for 1 hour in a thermostatic bath at 70°C. After 1 hour, a neutralizing solution (36% hydrochloric acid: 3.14 g, purified water: 3.14 g) was loaded in, the mixture was stirred, and the sample was washed 3 times with 8000 mL of purified water using suction filtration to obtain particles having a charge capacity of 0.55 mmol/g. The porous cellulose microparticles obtained in this manner were labeled as (b).

After again obtaining particles with a charge capacity of 0.55 mmol/g, 6.66 g of the particles was loaded into the reaction mixture (purified water: 217.30 g, anhydrous sodium sulfate: 88.83 g, sodium dodecylsulfate: 0.016 g, 2-(diethylamino)ethyl chloride hydrochloride solution (50%): 2.325 g), which was then stirred. A reaction starting solution (48% aqueous sodium hydroxide solution: 1.930 g, purified water: 6.83 g) was then added and the mixture was gently stirred, after which reaction was conducted while stirring for 1 hour in a thermostatic bath at 70°C. After 1 hour, a neutralizing solution (36% hydrochloric acid: 3.14 g, purified water: 3.14 g) was loaded in, the mixture was stirred, and the sample was washed 3 times with 8000 mL of purified water using suction filtration to obtain particles having a charge capacity of 1.04 mmol/g. The porous cellulose microparticles obtained in this manner were labeled as (c).

After further obtaining particles with a charge capacity of 1.80 mmol/g, 6.66 g of the particles was loaded into the reaction mixture (purified water: 217.30 g, anhydrous sodium sulfate: 88.83 g, sodium dodecylsulfate: 0.016 g, 2-(diethylamino)ethyl chloride hydrochloride solution (50%): 11.08 g), which was then stirred. A reaction starting solution (48% aqueous sodium hydroxide solution: 5.33 g, purified water: 6.83 g) was then added and the mixture was gently stirred, after which reaction was conducted while stirring for 1 hour in a thermostatic bath at 70°C. After 1 hour, a neutralizing solution (36% hydrochloric acid: 3.14 g, purified water: 3.14 g) was loaded in, the mixture was stirred, and the sample was washed 3 times with 8000 mL of purified water using suction filtration to obtain particles having a charge capacity of 2.60 mmol/g. The porous cellulose microparticles obtained in this manner were labeled as (d).

Thus, four porous cellulose microparticles with different charge capacities were obtained.

[Example 1: Measurement of dry particle weight per swelled volume]

The dry particle weight (mg) per swelled volume (mL) was measured for the invention particles (a) and the comparison particles (1) to (4). The results are shown in Table 2 below.

Table 2]

| | Invention particles (a) | Comparison particles (1) | Comparison particles (2) | Comparison particles (3) | Comparison particles (4) |
|---|---|---|---|---|---|
| Dry particle weight per swelled volume (mg/mL) | 36 | 83 | 59 | 500 | 333 |

The invention particles had the smallest dry particle weight per swelled volume compared to the comparison particles, with notable water absorption and swelling even at a low weight. Since a notable difference was seen even compared to the comparison particles (1) which had similar mean pore sizes during swelling, this suggests that the crosslinked cellulose material and its structure were contributing.

[Example 2: Measurement of permissible water content C and free water ratio F]

In order to measure the ease with which liquid or gas permeated the porous particles, the permissible water content C and free water ratio F of the invention particles (a) to (d) and the comparison particles (1) to (4) were measured. The results are shown in Table 3 below.

[Table 3]

| | Invention particles | | | | Comparison particles | | | |
|---|---|---|---|---|---|---|---|---|
| | (b) | (c) | (a) | (d) | (1) | (2) | (3) | (4) |
| Charge capacity (mmol/g) | 0.55 | 1.04 | 1.8 | 2.6 | 0.52 | 1.53 | Below detection limit | 3.2 |
| Permissible water content C per 100 mg particles (g) | 2.080 | 2.600 | 2.980 | 3.380 | 1.190 | 1.851 | 0.216 | 0.261 |
| Bound water content E per 100 mg particles (g) | 0.311 | 0.322 | 0.330 | 0.339 | 0.404 | 0.932 | 0.057 | 0.084 |
| Free moisture content F ((C-E) /E) | 5.7 | 7.1 | 8.0 | 9.0 | 1.9 | 1.0 | 2.8 | 2.1 |

Comparison of the permissible water contents indicated that when packed into an empty column, the invention particles retained considerably more water than the comparison particles (1) to (4). On the other hand, since the water was easily freed from the particles and exchanged with gas by gentle centrifugal force of 1000 G for 1 minute, this indicated the physical property of a very high free water ratio. In other words, the invention particles exhibited very easy permeation of liquid or gas due to their high porosity.

[Example 3: Measurement of *E. coli* adsorption performance by vortex method]

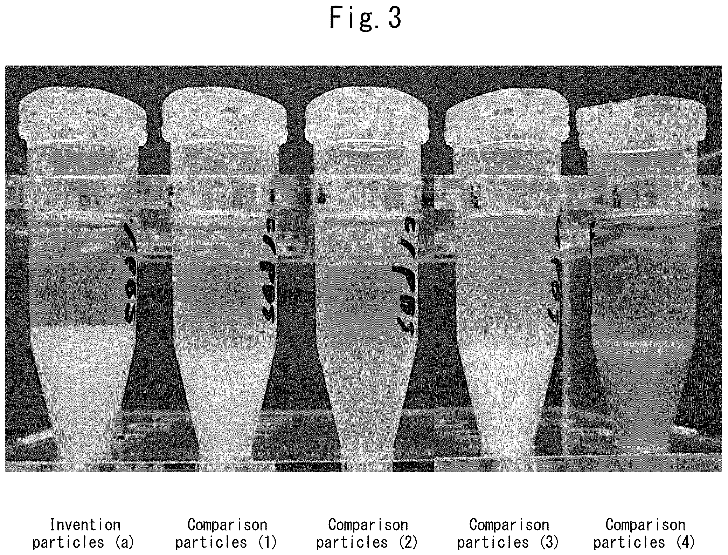

Based on the dry particle weight per swelled volume determined in Example 1, the invention particles (a) and the comparison particles (1) to (4) were weighed out to uniform swelled particle volumes as shown in Table 4 (measuring the weight minus the water content for wet particles or suspended particles). After adding 10 mL of PBS(-) to each of the particles, the mixture was sterilized with an autoclave at 121°C for 20 minutes. This was followed by centrifugal separation at 1000 G for 1 minute, after which the supernatant liquid was removed by suction using an aspirator and 10 mL of sterilized PBS(-) was added. After twice carrying out a procedure of washing the particles and charge equilibration, the mixture was adjusted upward to a final liquid volume of 4 mL. Fig. 3 shows prepared suspensions of the invention particles (a) and comparison particles (1) to (4).

[Table 4]

| | Invention particles (a) | Comparison particles (1) | Comparison particles (2) | Comparison particles (3) | Comparison particles (4) |
|---|---|---|---|---|---|
| Dry particle weight (mg) | 40 | 93 | 66 | 560 | 373 |

(continued)

|  | Invention particles (a) | Comparison particles (1) | Comparison particles (2) | Comparison particles (3) | Comparison particles (4) |
|---|---|---|---|---|---|
| Swelled particle volume (mL) | 1.12 | 1.12 | 1.12 | 1.12 | 1.12 |
| Final liquid volume of particle suspension (mL) | 4 | 4 | 4 | 4 | 4 |
| Final concentration (mg/mL) | 10 | 23 | 16 | 140 | 93 |

A 0.2 mL portion of each uniformly dispersed particle suspension (corresponding to a swelled particle volume of 0.056 mL) was weighed out into a 2 mL sterile sampling tube, and after adding 0.8 mL of a sample of the *E. coli* solution prepared in (8) above diluted 1e4-fold or 1e6-fold, the mixture was vortexed for 30 seconds or 2 minutes using a TUBE MIXER (MT-360 eccentric oscillation system, TOMY Co.) at maximum power. This was followed by centrifugal separation at 400 G for 1 minute, and after diluting the supernatant liquid with physiological saline to a dilution ratio of K (factor) of the concentration suitable for plate smear culture, 100 μL was inoculated into standard agar medium (8-MR22, Pourmedia). Culturing was carried out at 35°C for 48 hours, and the number of colonies L (cfu/mL) was counted. The same procedure was also carried out using 0.2 mL of physiological saline instead of the particle suspension, and the number of colonies M (cfu/mL) was counted. The adsorption and adsorption rate of *E. coli* per dry particle weight were calculated by the following formulas (5) and (6), respectively. The dry particle weights used in the formulas are those listed in Table 4.

Adsorption per dry particle weight N (number/mg) = K × (M - L)(dry particle weight/20)          Formula (5)

$$\text{Adsorption rate (\%)} = (M - L)/M \times 100 \quad \text{Formula (6)}$$

The results are shown in Table 5.

[Table 5]

| Vortex time | Sample | Measured property | Invention particles (a) | Comparison particles (1) | Comparison particles (2) | Comparison particles (3) | Comparison particles (4) |
|---|---|---|---|---|---|---|---|
| 30 sec | 1e4-fold diluted *E. coli* solution (1.3e5 cfu/mL) | Adsorption rate (%) | 81 | <1 | <1 | <1 | 17 |
|  |  | Adsorption N per dry particle weight (number/mg) | 5e4 | Unable to calculate | Unable to calculate | Unable to calculate | 1e3 |
|  | 1e6-fold diluted *E. coli* solution (2.0e3 cfu/mL) | Adsorption rate (%) | 84 | 42 | 38 | 84 | 53 |
|  |  | Adsorption N per dry particle weight (number/mg) | 9e2 | 2e2 | 3e2 | 6e1 | 7e1 |

(continued)

| Vortex time | Sample | Measured property | Invention particles (a) | Comparison particles (1) | Comparison particles (2) | Comparison particles (3) | Comparison particles (4) |
|---|---|---|---|---|---|---|---|
| 2 min | 1e4-diluted *E. coli* solution (0.7e5 cfu/mL) | Adsorption rate (%) | 84 | 16 | <1 | 12 | 76 |
| | | Adsorption N per dry particle weight (number/mg) | 3e4 | 2e3 | Unable to calculate | 3e2 | 3e3 |
| | 1e6-diluted *E. coli* solution (0.8e3 cfu/mL) | Adsorption rate (%) | 74 | 8.3 | 1.7 | 90 | 74 |
| | | Adsorption N per dry particle weight (number/mg) | 3e2 | 1e1 | 4e0 | 3e1 | 3e1 |

The invention particles (a) maintained a high adsorption rate across a wide range of *E. coli* concentrations (1e3 to 1e5 cfu/mL), compared to the comparison particles (1) to (4). For high adsorption capacity it is necessary for *E. coli* to be adsorbed throughout the interiors of the particles, and the particles must therefore have pore sizes of sufficient size, but since the invention particles (a) had a considerably high adsorption rate even compared to the comparison particles (1) which had a similar mean pore size during swelling, this indicated that the invention particles (a) had high permeability due not only to the pore sizes but also to the high porosity, thereby exhibiting highly efficient adsorption of *E. coli* that was not possible with conventional particles.

Moreover, the comparison particles (3) and (4), which had adsorption rates of 70% or greater with 2 minutes of vortex, had a notably lower adsorption rate at 30 seconds, whereas the invention particles (a) exhibited the same performance even after 30 seconds, demonstrating that the aforementioned structural features also contribute to rapid adsorption.

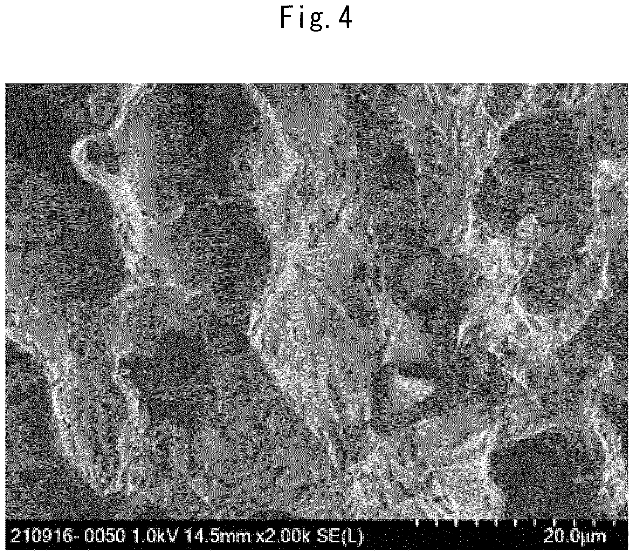

Fig. 4 shows an example of an SEM photograph of porous cellulose microparticles after E. *coli* adsorption.

Since the invention particles (a) exhibited highly efficient adsorption performance compared to comparison particles (1) to (4), which had uniform swelled particle volumes even though having high porosity, it was thereby possible to obtain a high-density mass of microorganisms with considerably high adsorption per dry particle weight N (number/mg).

[Example 4: Measurement of *E. coli* adsorption performance by column method]

A 0.2 mL portion of the uniformly dispersed particle suspension prepared in Example 3 was packed into an empty column (Presh-SPE$^R$, Aisti Co., Ltd.), and the excess PBS(-) was removed by aspiration through the outlet to prepare a particle-packed column. A 10 mL portion of a sample obtained by 1e3-fold dilution of the *E. coli* solution prepared in (8) above (hereunder referred to as "1e3-fold diluted *E. coli* solution" or "E. *coli* sample") was passed through the column at 100 μL/sec using a disposable syringe, and the solution passing through the column (hereunder referred to as "flow-through") was collected. Next, 1 mL of physiological saline was passed through the column to wash the E. coli-adsorbed column, and 10 mL of air was further aerated through to remove the free water. The *E. coli* sample and the flow-through were diluted with physiological saline to a dilution ratio producing a concentration suitable for plate smear culture, and then 100 μL was inoculated into standard agar medium (8-MR22, Pourmedia), culturing was carried out at 35°C for 48 hours, and the number of colonies was counted. The adsorption T and adsorption rate S of *E. coli* per dry particle weight were calculated by the following formulas (7) and (8), respectively. The dry particle weights used in the formulas are those listed in Table 4.

*E.coli* sample: Cell concentration O (cfu/mL) = Inoculated colony count (cfu/mL) × dilution ratio Q Flow-through: Cell concentration P (cfu/mL) = Inoculated colony count (cfu/mL) × dilution ratio R Adsorption per dry particle weight S (number/mg) = (O - P) × 10 (mL)/(dry particle weight/20)

$$\text{Adsorption rate T }(\%) = (O - P)/O \times 100 \qquad \text{Formula (8)}$$

The results are shown in Table 6 below.

[Table 6]

| Sample | Measured property | Invention particles (a) | Comparison particles (1) | Comparison particles (2) | Comparison particles (3) | Comparison particles (4) |
|---|---|---|---|---|---|---|
| 1e3-diluted E. *coli* solution (3e6 cfu/mL) | Adsorption rate T (%) | 96 | 12 | <1 | <1 | 39 |
| | Adsorption S per dry particle weight (number/mg) | 2e7 | 9e5 | Unable to calculate | Unable to calculate | 7e5 |

In the column method as well, the invention particles (a) had a high adsorption rate T compared to the comparison particles (1) to (4), and could exhibit high adsorption performance whether with the vortex method (method of contacting the particles and microorganisms by uniform dispersion) or the column method (method of contacting the particles and microorganism by solid phase separation). It was confirmed that after *E. coli* adsorption in the column method, the particles could be washed and the free water remaining on the particles could be easily removed by aeration.

[Example 5: Measurement of adsorption performance of microorganisms other than *E. coli* by column method]

The adsorption S and adsorption rate T of microorganisms per dry particle weight were each calculated for the *E. coli, Staphylococcus aureus* and *E. coli* T1 phage as a virus prepared in (8) above, by the same procedure as Example 3 and Example 4. For E. *coli* T1 phage measurement, however, 10 μL of diluted solution was mixed with 30 μL of 1e9 cfu/mL JM109 *E. coli* and 5 mL of soft agar, the mixture was solidified on standard agar medium, and the number of plaques cultured at 35°C for 24 hours was counted. The adsorption rate T and the adsorption per dry microparticle weight S of the *E. coli* T1 phage were counted by replacing cfu/mL with pfu/mL. The results are shown in Table 7 below.

[Table 7]

| Microorganism type | Gram-negative bacterium | Gram-positive bacterium | Virus |
|---|---|---|---|
| | *E. coli* | *S. aureus* | *E. coli* T1 phage |
| Strain | ATCC No.25922 | ATCC No.25923 | NBRC 20001 |
| Microorganism sample concentration O | 1e6 (cfu/mL) | 0.9e6 (cfu/mL) | 0.7e6 (pfu/mL) |
| Adsorption rate T (%) | 99 | 97 | 100 |
| Adsorption S per dry particle weight (number/mg) | 5e6 | 4e6 | 3e6 |

The results shown in Table 7 demonstrated that adsorption at high efficiency was possible using the invention particles (a), regardless of whether the type of bacterium was gram-negative *E. coli* or gram-positive *Staphylococcus aureus*. Similar adsorption power was also exhibited when using viruses as the microorganisms instead of bacteria.

[Example 6: Method for collection of cells from *E. coli* adsorbed particles (high-density mass of microorganisms) obtained by column method, and obtaining condensate of *E. coli*]

After passing 1 mL of 1 M magnesium chloride as an eluent through the E. coli-adsorbed column from which the free water had been removed, obtained as the final product in Example 4, the eluent flow-through was collected. The flow-through was diluted with physiological saline to a dilution ratio V producing a concentration suitable for plate smear culture, and then 100 μL was inoculated into standard agar medium (8-MR22, Pourmedia), culturing was carried out at 35°C for 48 hours, and the number of colonies was counted. The concentration rate W for *E. coli* was calculated by the following formula (9).

Eluent flow-through: Cell concentration U (cfu/mL) = Inoculated colony count (cfu/mL) $\times$ dilution ratio V

Concentration rate W (factor) = Eluent flow-through cell concentration U/*E. coli* sample cell concentration O

The results are shown in Table 8 below.

[Table 8]

| Sample | Measured property | Invention particles (a) | Comparison particles (1) | Comparison particles (2) | Comparison particles (3) | Comparison particles (4) |
|---|---|---|---|---|---|---|
| 1e3-diluted *E. coli* solution (3e6 cfu/mL) | Concentration rate W (factor) | 2.7 | <0.1 | 0.9 | <0.1 | 0.3 |

It was shown that the invention particles (a) had one or more concentration rates, and that the cells can be concentrated by passing through eluent.

[Example 7: Measurement of adsorption rate T (%) and concentration rate W (factor) for invention particles (a) to (d) having different charge capacities]

The adsorption rate T (%) and concentration rate W (factor) for the four invention particles (a) to (d) having different charge capacities were measured by the same procedure as in Example 4 and Example 5, with the results shown in Table 9.

[Table 9]

| | Invention particles (b) | Invention particles (c) | Invention particles (a) | Invention particles (d) |
|---|---|---|---|---|
| Charge capacity (mmol/g) | 0.55 | 1.04 | 1.8 | 2.6 |
| *E. coli* sample concentration O (cfu/mL) | 1e6 | 1e6 | 1e6 | 1e6 |
| Adsorption rate T (%) | 51 | 96 | 96 | 98 |
| Concentration rate W (factor) | 0.7 | 6.8 | 2.7 | 4.0 |

The results in Table 9 demonstrated that the adsorption rate was 50% or greater when the charge capacity of the porous cellulose microparticles was 0.55 to 2.6 mmol/g. It was shown that for the concentration rate W, the charge capacity is preferably 1.04 to 2.6 mmol when the eluent is 1 M magnesium chloride.

[Example 8: Measurement of adsorption rate T (%) and concentration rate W (factor) of invention particles (a) based on cell concentration, flow volume and eluent volume with different *E. coli* samples]

The adsorption rate T (%) and concentration rate W (factor) for different *E. coli* samples based on cell concentration O, flow volume and eluent volume were measured using a column packed with 0.2 mL of a particle suspension of the invention particles (a) (2 mg by dry particle weight), by the same procedure as in Example 4 and Example 5, with the results shown in Table 10. The eluent volumes in Table 10 are the volumes (mL) of 1 M magnesium chloride passing through as eluent.

Table 10

| | | | | | | |
|---|---|---|---|---|---|---|
| *E. coli* sample concentration O (cfu/mL) | 1E6 | 1E6 | 1E1 | 1E3 | 1E7 | 1E8 |
| *E. coli* sample flow volume (mL) | 10 | 10 | 100 | 1000 | 10 | 10 |
| Eluent volume (mL) | 1 | 0.1 | 1 | 1 | 1 | 1 |
| Adsorption rate T (%) | 96 | 98 | 100 | 100 | 94 | 46 |
| Concentration rate W (factor) | 2.7 | 12 | 39 | 284 | 2.6 | 0.6 |

The results in Table 10 demonstrated that a column packed with 0.2 mL of particle suspension of invention particles (a) (corresponding to 2 mg as dry particle weight, and 0.056 mL as swelled particle volume) can pass through with a 10 to 1000 mL flow volume of *E. coli* sample and can be collected with an eluent volume of 0.1 to 1 mL, indicating that a high adsorption rate T and concentration rate W similar to Example 4 and Example 5 can be maintained within this range. The column was also able to adsorb up to a cell concentration O of 1e8 cfu/mL in the *E. coli* sample, with a particularly high adsorption rate T being obtained with a concentration of 1e1 to 1e7 cfu/mL.

INDUSTRIAL APPLICABILITY

With the concentration method and concentration vessel of the invention it is possible to concentrate a microorganism using specific porous cellulose microparticles having a high porosity and dehydration rate as indicated by the permissible water content and free water ratio, to allow high adsorption of the microorganism per dry weight of the particles and obtain a high-density mass of the microorganism, and also to collect the microorganism at high-concentration from the high-density mass of the microorganism. The concentration method and concentration vessel of the invention can therefore be suitably used for microbial examination.

**Claims**

1.  A microorganism concentration method wherein a microorganism in a sample liquid comprising the microorganism in a solvent is concentrated using porous cellulose microparticles having numerous interior pores, the method including the following steps:

    a step of contacting the sample liquid with the porous cellulose microparticles to adsorb the microorganism in the sample liquid onto the surfaces of the porous cellulose microparticles, and
    a liquid removal step in which the solvent of the sample liquid is removed to concentrate the microorganism on the surface and/or in the pores of the porous cellulose microparticles;

    wherein when 100 mg of the dry porous cellulose microparticles is swelled with PBS(-), the permissible water content C is 1.9 g or greater, as the mass of PBS(-) in the porous cellulose microparticles in a swelled state such that PBS(-) does not fall by its own weight, and the free water ratio ((C - E)/E) is 2.9 or greater, as calculated by dividing the dehydration amount (C - E) (obtained by subtracting the mass E of the PBS(-) after centrifugation of the swelled porous cellulose microparticles for 1 minute at 1000 G from the permissible water content C) by E.

2.  The method according to claim 1, which further includes the following step: a step in which, after the liquid removal step, the microorganism concentrated on the surface and/or in the pores of the porous cellulose microparticles using an eluent is recovered in the eluent.

3.  The method according to claim 1 or 2, which further includes a step between each of the steps, in which the porous cellulose microparticles are washed with a liquid or gas.

4.  The method according to claim 1 or 2, wherein the mean particle size of the porous cellulose microparticles is 100 $\mu$m to 1000 $\mu$m.

5. The method according to claim 1 or 2, wherein the mean pore size of the pores in the porous cellulose microparticles is 1 $\mu$m to 100 $\mu$m.

6. The method according to claim 1 or 2, wherein the porous cellulose microparticles form a continuous pore structure with adjacent pores mutually communicating by openings in the membranes dividing them.

7. The method according to claim 1 or 2, wherein the surface area of the porous cellulose microparticles per 1 g of dry particles (the specific surface area) is 0.3 $m^2$/g to 4.4 $m^2$/g.

8. The method according to claim 1 or 2, wherein the cellulose composing the porous cellulose microparticles has a cationic functional group, and the charge capacity is 0.5 mmol/g to 5.0 mmol/g.

9. The method according to claim 8, wherein the cationic functional group is at least one selected from the group consisting of primary amino, secondary amino and tertiary amino groups.

10. The method according to claim 9, wherein the cationic functional group is an N,N-diethylaminoethyl (DEAE) group.

11. The method according to claim 1 or 2, wherein the cellulose composing the porous cellulose microparticles is crosslinked between the molecules.

12. The method according to claim 1 or 2, wherein the cellulose composing the porous cellulose microparticles is made of copper ammonia regenerated cellulose.

13. The method according to claim 1 or 2, wherein the microorganism is a bacterium or virus.

14. A concentration vessel to be used in a method in which a microorganism in a sample liquid comprising the microorganism in a solvent is concentrated using porous cellulose microparticles having numerous interior pores, wherein the concentration vessel comprises:

    a cylindrical body having an inlet opening and an outlet opening;
    a filter provided on the outlet opening side; and
    porous cellulose microparticles filled into the spaces on the filter;

    wherein

    the sample liquid supplied through the inlet opening side is contacted with the porous cellulose microparticles, and the microorganism in the sample liquid is adsorbed onto the surfaces of the porous cellulose microparticles while the solvent of the sample liquid is removed from the outlet opening side, thereby concentrating the microorganism on the surface and/or in the pores of the porous cellulose microparticles, and
    when 100 mg of the dry porous cellulose microparticles is swelled with PBS(-), the permissible water content C is 1.9 g or greater, as the mass of PBS(-) in the porous cellulose microparticles in a swelled state such that PBS(-) does not fall by its own weight, and the free water ratio ((C - E)/E) is 2.9 or greater, as calculated by dividing the dehydration amount (C - E) (obtained by subtracting the mass E of the PBS(-) after centrifugation of the swelled porous cellulose microparticles for 1 minute at 1000 G from the permissible water content C) by E.

15. The concentration vessel according to claim 14, wherein the mean particle size of the porous cellulose microparticles is 100 $\mu$m to 1000 $\mu$m.

16. The concentration vessel according to claim 14 or 15, wherein the mean pore size of the pores in the porous cellulose microparticles is 1 $\mu$m to 100 $\mu$m.

17. The concentration vessel according to claim 14 or 15, wherein the porous cellulose microparticles form a continuous pore structure with adjacent pores mutually communicating by openings in the membranes dividing them.

18. The concentration vessel according to claim 14 or 15, wherein the surface area of the porous cellulose microparticles per 1 g of dry particles (the specific surface area) is 0.3 $m^2$/g to 4.4 $m^2$/g.

19. The concentration vessel according to claim 14 or 15, wherein the cellulose composing the porous cellulose microparticles has a cationic functional group, and the charge capacity is 0.5 mmol/g to 5.0 mmol/g.

20. The concentration vessel according to claim 19, wherein the cationic functional group is at least one selected from the group consisting of primary amino, secondary amino and tertiary amino groups.

21. The concentration vessel according to claim 20, wherein the cationic functional group is an N,N-diethylaminoethyl (DEAE) group.

22. The concentration vessel according to claim 14 or 15, wherein the cellulose composing the porous cellulose microparticles is crosslinked between the molecules.

23. The concentration vessel according to claim 14 or 15, wherein the cellulose composing the porous cellulose microparticles is made of copper ammonia regenerated cellulose.

24. The concentration vessel according to claim 14 or 15, wherein the microorganism is a bacterium or virus.

25. A microorganism concentration method wherein a microorganism in a sample liquid comprising the microorganism in a solvent is concentrated using porous cellulose microparticles having numerous interior pores, the method including the following steps:

a step of contacting the sample liquid with the porous cellulose microparticles in a concentration vessel comprising a cylindrical body having an inlet opening and an outlet opening; a filter provided on the outlet opening side; and porous cellulose microparticles filled into the spaces on the filter, to adsorb the microorganism in the sample liquid onto the surfaces of the porous cellulose microparticles, and

a liquid removal step in which aeration is carried out and the solvent of the sample liquid is removed to concentrate the microorganism on the surface and/or in the pores of the porous cellulose microparticles;

wherein when 100 mg of the dry porous cellulose microparticles is swelled with PBS(-), the permissible water content C is 1.9 g or greater, as the mass of PBS(-) in the porous cellulose microparticles in a swelled state such that PBS(-) does not fall by its own weight, and the free water ratio ((C - E)/E) is 2.9 or greater, as calculated by dividing the dehydration amount (C - E) (obtained by subtracting the mass E of the PBS(-) after centrifugation of the swelled porous cellulose microparticles for 1 minute at 1000 G from the permissible water content C) by E.

26. The microorganism concentration method according to claim 25, wherein the aeration is aeration with air using a syringe from the inlet opening part of the concentration vessel.

27. The microorganism concentration method according to claim 26, wherein the volume of the air is 0.5 mL to 1 mL per 1 mg of the porous cellulose microparticles.

# Fig.1

Crosslinked cellulose
Scanning electron microscope
(wet)

Crosslinked gelatin
Contrast CT
(wet)
(NPL 1)

Crosslinked dextran
Contrast CT
(wet)
(NPL 1)

Crosslinked polystyrene
Micro CT
(dry)
(NPL 1)

Activated magnesium silicate
Scanning electron microscope
(wet)
(NPL 2)

NPL 1    Sci Rep. 2021; 11: 2819
NPL 2    J Anal Methods Chem.
         2015; 2015: 562780.

# Fig. 2

Dry particles 100 mg

Swelling
PBS (−)

Swelled particles

Column packing

Column

Fall by gravity

Centrifugal separation
1,000 G, 1 min

$$\text{Free water ratio } F = \frac{\text{Free water content } C-E}{\text{bound water content } E}$$

Swelled particles

Permissible water content C

Gravity

Free water content C−E

Mass E of PBS(−) remaining in particles after centrifugation (bound water content)

Resistance

Free water content C−E

Centrifugal force

# Fig. 3

| Invention<br>particles (a) | Comparison<br>particles (1) | Comparison<br>particles (2) | Comparison<br>particles (3) | Comparison<br>particles (4) |

EP 4 446 405 A1

Fig. 4

210916- 0050 1.0kV 14.5mm x2.00k SE(L)                    20.0μm

# Fig. 5

Inlet opening

Porous
cellulose
microparticles

Filter

Outlet opening

## INTERNATIONAL SEARCH REPORT

| International application No. |
|---|
| **PCT/JP2022/044415** |

**A. CLASSIFICATION OF SUBJECT MATTER**

*C12N 1/20*(2006.01)i; *C12M 1/26*(2006.01)i; *C12N 7/02*(2006.01)i; *B01J 20/24*(2006.01)n; *B01J 20/28*(2006.01)n
FI:   C12N1/20 Z; C12N7/02; C12M1/26; B01J20/24 B; B01J20/28 Z

According to International Patent Classification (IPC) or to both national classification and IPC

**B. FIELDS SEARCHED**

Minimum documentation searched (classification system followed by classification symbols)

C12N1/20; C12M1/26; C12N7/02; B01J20/24; B01J20/28

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2015/029790 A1 (JNC CORPORATION) 05 March 2015 (2015-03-05) claims, examples, tables 1-4 | 1-27 |
| A | JP 2016-195589 A (KANEKA CORP) 24 November 2016 (2016-11-24) claims | 1-27 |
| A | JP 2011-220992 A (JNC CORPORATION) 04 November 2011 (2011-11-04) claims | 1-27 |
| A | JP 2019-194547 A (JNC CORPORATION) 07 November 2019 (2019-11-07) claims, paragraph [0024] | 1-27 |
| P, A | JP 2022-63983 A (ASAHI KASEI CORP) 25 April 2022 (2022-04-25) claims, paragraph [0026], examples | 1-27 |

☐ Further documents are listed in the continuation of Box C.      ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **02 February 2023** | **14 February 2023** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

<p style="text-align:center"><b>INTERNATIONAL SEARCH REPORT</b><br>
Information on patent family members</p>

International application No.

**PCT/JP2022/044415**

| Patent document cited in search report | Publication date (day/month/year) | Patent family member(s) | Publication date (day/month/year) |
|---|---|---|---|
| WO 2015/029790 A1 | 05 March 2015 | US 2016/0200835 A1 claims, examples, tables 1-4 EP 3042925 A1 | |
| JP 2016-195589 A | 24 November 2016 | (Family: none) | |
| JP 2011-220992 A | 04 November 2011 | US 2010/0330119 A1 claims EP 2266675 A2 | |
| JP 2019-194547 A | 07 November 2019 | (Family: none) | |
| JP 2022-63983 A | 25 April 2022 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5972174 B
- JP 4889263 B
- JP 2543766 B
- JP 4161167 B
- JP 2019194547 A
- JP 4091142 A